**Europäisches Patentamt**

**European Patent Office**

**Office européen des brevets**

(11) Veröffentlichungsnummer: **0 344 585**
**A1**

# (12) EUROPÄISCHE PATENTANMELDUNG

(21) Anmeldenummer: **89109311.4**

(22) Anmeldetag: **24.05.89**

(51) Int. Cl.⁴: **C07C 49/255 , C07C 45/57 , C07C 45/87**

(30) Priorität: **28.05.88 DE 3818244**

(43) Veröffentlichungstag der Anmeldung:
**06.12.89 Patentblatt 89/49**

(84) Benannte Vertragsstaaten:
**BE CH DE FR GB LI**

(71) Anmelder: **HOECHST AKTIENGESELLSCHAFT**
**Postfach 80 03 20**
**D-6230 Frankfurt am Main 80(DE)**

(72) Erfinder: **Günther, Klaus, Dr.**
**Waldstrasse 5**
**D-6239 Eppstein Taunus(DE)**

(54) **Acetale sowie Verfahren zur deren Herstellung.**

(57) Die Erfindung betrifft Acetale der Formel $CH_3\text{-}CO\text{-}CH = C(OR)_2$, wobei $R = -(CH_2)_n\text{-}OR'$ mit $n = 2$ oder $3$ und $R' = C_1\text{-}C_5$-Alkyl ist.

Weiter betrifft die Erfindung ein Verfahren zur Herstellung von Acetalen der Formel $CH_3\text{-}CO\text{-}CH = C(OR)_2$, wobei $R = C_1\text{-}C_{12}$-Alkyl oder $R = -(CH_2)_n\text{-}OR'$ mit $n = 2$ oder $3$ und $R' = C_1\text{-}C_5$-Alkyl ist. Das Verfahren besteht darin, daß man Diketen in Gegenwart eines sauren Katalysators mit einem Alkohol der Formel ROH umsetzt, wobei R die angegebene Bedeutung hat.

EP 0 344 585 A1

## Acetale sowie Verfahren zu deren Herstellung

Die Erfindung betrifft ein Verfahren zur Herstellung von Acetalen der Formel (I)

$CH_3\text{-}CO\text{-}CH = C(OR)_2$     (I)

wobei R $= C_1\text{-}C_{12}$-Alkyl oder R $= - (CH_2)_n\text{-}OR'$ mit n $= 2$ oder 3 und R' $= C_1\text{-}C_5$-Alkyl ist. Außerdem betrifft die Erfindung die neuen Acetale der Formel (I) mit R $= - (CH_2)_n\text{-}OR'$, wo n und R' die genannte Bedeutung haben.

Nach Literaturangaben wird Acetylketendiethylacetal $CH_3\text{-}CO\text{-}CH = C(OC_2H_5)_2$, also die Verbindung der Formel (I) mit R $=$ Ethyl, durch Umsetzung von Ketendiethylacetal mit Acetylchlorid hergestellt, wobei jedoch erhebliche Mengen an Nebenprodukten entstehen (Mc Elvain, Mc Shane, J. Am. Chem. Soc. 74, 1952, Seiten 2662, 2666). Jedoch ist die Ausgangssubstanz Ketendiethylacetal schwer zugänglich; seine Synthese weist viele Reaktionsstufen auf, die meist mit unbefriedigender Ausbeute verlaufen. Demgemäß ist dieses Verfahren zur Herstellung von Acetylketendiethylacetal im größeren Maßstab wenig geeignet. Weiterhin ist in der Literatur nur noch die Existenz von Acetylketendimethylacetal erwähnt (Chem. Abstr., Band 55, Nr. 23, 1961, 23335). Acetylketendialkylacetale, die höhere Alkylgruppen als Methyl und Ethyl enthalten, werden in der Literatur nicht beschrieben.

Angaben über die Herstellung oder die Eigenschaften von Acetylketendialkoxyalkylacetalen, also Verbindungen der Formel (I) mit R $= - (CH_2)_n\text{-}OR'$ mit n $= 2$ oder 3 und R' $= C_1\text{-}C_5$-Alkyl sind in der Literatur überhaupt nicht zu finden.

Kürzlich wurde nun ein einfaches Verfahren gefunden, das es gestattet, Acetylketendialkylacetale $CH_3\text{-}CO\text{-}CH = C(OR)_2$ mit R $= C_1\text{-}C_{12}$-Alkyl zu gewinnen (Deutsche Patentanmeldung P 3 644 661.0).

Gemäß diesem Verfahren werden Acetessigsäurealkylester der Formel $CH_3\text{-}CO\text{-}CH_2\text{-}COOR^1$ in Gegenwart eines sauren Katalysators mit einem Alkohol der Formel $R^2OH$ umgesetzt und ergeben dabei Acetylketendialkylacetale der Formel $CH_3\text{-}CO\text{-}CH = C(OR^2)_2$, wobei $R^2$ ein Alkylrest mit 1 bis 12 C-Atomen sein kann und $R^2OH$ mit $R^1OH$ identisch ist oder höher siedet als $R^1OH$.

Die vorliegende Erfindung geht dagegen von Diketen statt von Acetessigsäurealkylestern aus.

Ein Gegenstand der Erfindung ist ein Verfahren zur Herstellung von Acetalen der Formel (I)

$CH_3\text{-}CO\text{-}CH = C(OR)_2$     (I)

wobei R $= C_1\text{-}C_{12}$-Alkyl oder R $= - (CH_2)_n\text{-}OR'$ mit n $= 2$ oder 3 und R' $= C_1\text{-}C_5$-Alkyl ist, dadurch gekennzeichnet, daß man Diketen in Gegenwart eines sauren Katalysators mit einem Alkohol der Formel ROH umsetzt, wobei R die angegebene Bedeutung hat.

Ein weiterer Gegenstand der Erfindung sind Acetale der Formel (I)

$CH_3\text{-}CO\text{-}CH = C(OR)_2$     (I)

wobei R $= - (CH_2)_n\text{-}OR'$ mit n $= 2$ oder 3 und R' $= C_1\text{-}C_5$-Alkyl ist.

Bei dem erfindungsgemäßen Verfahren bildet sich neben dem gewünschten Acetal eine gewisse Menge Acetessigsäureester der Formel $CH_3\text{-}CO\text{-}CH_2\text{-}COOR$, wobei R $= C_1\text{-}C_{12}$-Alkyl oder R $= - (CH_2)_n\text{-}OR'$, mit n $= 2$ oder 3 und R' $= C_1\text{-}C_5$-Alkyl ist. Das gewonnene Reaktionsgemisch läßt sich durch fraktionierte Destillation leicht trennen, wobei das Acetal als am höchsten siedende Komponente zuletzt überdestilliert. Die Fraktion, die den Acetessigsäureester enthält, kann erneut mit den entsprechenden Alkoholen zu Acetal umgesetzt werden.

Als Alkohole ROH kommen für die Herstellung der Acetale der Formel $CH_3\text{-}CO\text{-}CH = C(OR)_2$, R $= C_1\text{-}C_{12}$-Alkyl, gesättigte primäre und sekundäre Alkohole mit 1 bis 12 C-Atomen, vorzugsweise 3 bis 6 C-Atomen, in Frage, wobei die C-Kette geradkettig oder verzweigt sein kann; vorzugsweise verwendet man gesättigte primäre Alkohole. Für die Herstellung der Acetale der Formel $CH_3\text{-}CO\text{-}CH = C(OR)_2$ mit R $= - (CH_2)_n\text{-}OR'$, wobei n $= 2$ oder 3 und R' $= C_1\text{-}C_5$-Alkyl ist, werden als Alkohole Ethylen- bzw. Propylenglykolmonoalkylether $R'O\text{-}(CH_2)_n\text{-}OH$, n $= 2$ oder 3, eingesetzt, wobei die Alkylgruppe R' 1 bis 5 C-Atome aufweist und geradkettig oder verzweigt sein kann. Vorzugsweise ist dabei n $= 2$ und R' $= C_1\text{-}C_3$-Alkyl.

Vorzugsweise setzt man pro Mol Diketen 1,5 bis 6 Mol ROH ein. Die Reaktionstemperatur sollte etwa 110 bis 170 °C betragen, vorzugsweise 130 bis 160 °C, um eine hinreichend hohe Reaktionsgeschwindigkeit zu erzielen. Bei Einsatz der relativ niedrig siedenden Alkohole ROH mit 1 bis 3 C-Atomen hat es sich als zweckmäßig erwiesen, die erforderliche Reaktionstemperatur von mindestens 110 °C dadurch zu erreichen, daß man die Reaktion unter geeignet erhöhtem Druck durchführt.

Als Katalysatoren benutzt man saure Komponenten. Bevorzugt kommen Protonensäuren, wie Schwefelsäure, p-Toluolsulfonsäure oder Phosphorsäure zum Einsatz. Auch saure Ionenaustauscher sowie Molekularsiebe sind geeignet. Der Konzentrationsbereich für den eingesetzten Katalysator liegt bei 0,005 bis 5 Gew.-%. Als sehr günstig hat sich bei Verwendung von konzentrierter Schwefelsäure eine Konzentration von 0,05 bis 0,2 Gew.-% erwiesen.

Für einen hinreichend schnellen Ablauf der Reaktion ist es erforderlich, das entstehende Reaktionswasser möglichst rasch und vollständig aus dem System zu entfernen. Grundsätzlich können dafür alle geeigneten Methoden zur Wasserentfernung aus Reaktionsgemischen, wie etwa die azeotrope Destillation unter Verwendung eines Schleppmittels, angewendet werden. Bei dem erfindungsgemäßen Verfahren hat es sich als ausreichend erwiesen, den Wasseraustrag bei der Reaktion dadurch zu unterstützen, daß man einen leichten Stickstoffstrom durch das Reaktionsgemisch strömen läßt und/oder das Reaktionswasser zusammen mit Überschußalkohol austrägt.

Im technischen Maßstab kann die Herstellung der Acetale nach dem erfindungsgemäßen Verfahren sowohl diskontinuierlich im Chargenbetrieb als auch kontinuierlich in einer geeigneten Apparateanordnung erfolgen.

Bei der diskontinuierlichen Gewinnung der Acetale wird in einer ersten Stufe Diketen mit überschüssigem Alkohol ROH umgesetzt und anschließend der Überschußalkohol zusammen mit dem gleichzeitig gebildeten Acetessigsäureester unter Vakuum abdestilliert, wobei das in dieser Stufe gebildete Acetal zurückbleibt. In einer zweiten Stufe läßt man den abdestillierten Acetessigsäureester mit überschüssigem Alkohol ROH wiederum unter Reaktionsbedingungen zum Acetal reagieren und destilliert danach den Überschußalkohol ROH zusammen mit dem unumgesetzten Acetessigsäureester unter Vakuum ab, wobei das in der 2. Stufe gebildete Acetal zurückbleibt. Zur Verbesserung der Ausbeute, bezogen auf das eingesetzte Diketen, kann man eventuell mit dem nach der zweiten Stufe abdestillierten Acetessigsäureester-Überschußalkohol-Gemisch eine nochmalige Umsetzung durchführen. Das in den einzelnen Schritten gebildete Acetal wird vereinigt und gemeinsam durch Vakuumdestillation gereinigt.

Die kontinuierliche Herstellung des Acetals kann in einer dafür geeigneten Apparatur auf recht elegante Weise erfolgen. In einen Reaktor speist man Diketen zusammen mit überschüssigem Alkohol ROH und Katalysator ein. Aus dem Reaktor wird ein Teil des Überschußalkohols (zusammen mit dem Reaktionswasser) dampfförmig abgezogen, das Reaktionsgemisch, bestehend aus Acetal, Acetessigsäureester und Alkohol ROH wird flüssig abgezogen. Das Reaktionsgemisch gelangt in eine Destillationskolonne, bei der über Kopf der überschüssige Alkohol ROH zusammen mit nicht umgesetztem Acetessigsäureester abdestilliert. Das Kopfprodukt wird in den Reaktor zurückgeführt. Aus dem Sumpf der Kolonne entnimmt man das unreine Acetal und führt es einer weiteren Kolonne zu, aus der es über Kopf als Reinprodukt abdestilliert. Im Sumpf dieser Kolonne reichern sich Katalysator und hochsiedende Rückstände an, die in geeigneter Weise entsorgt werden.

Die nach dem erfindungsgemäßen Verfahren herstellbaren Acetale sind Verbindungen, die als Ausgangsprodukte für Synthesen vielfältig eingesetzt werden können. Durch ihre Konstitution sind sie für Cyclisierungsreaktionen besonders geeignet.

So lassen sich zum Beispiel durch Umsetzung von Acetylketendimethylacetal mit 2-Hydrazinopyrimidin in einfacher Weise Alkoxypyrazole gewinnen (Chemical Abstracts, Vol. 79, 1973, Seite 388).

Es ist auch bekannt, daß Acetylketendimethylacetal und Acetylketendiethylacetal sich bei bestimmten Temperaturen als 1-Hydroxyvinylketenacetale verhalten und zum Beispiel an definierten Positionen substituierte Anthrachinone ergeben, die als Naturfarbstoffe von Bedeutung sind (J. Chem. Soc. Perkin Trans. I, 1976, 17, 1872-56).

Analoges gilt für die anderen erfindungsgemäß herstellbaren Acetale. Die Möglichkeit, durch das erfindungsgemäße Verfahren Acetylketendialkylacetale bzw. Acetylketendialkoxyalkylacetale in größerem Umfang günstig herstellen zu können, eröffnet eine Vielzahl von synthetischen Möglichkeiten.

Die folgenden Beispiele sollen die Erfindung erläutern. Die Prozentangaben sind Gewichtsprozente, falls nicht anders spezifiziert.

**Beispiel 1**

In einen 1 l-Vierhalskolben, der mit Rührer, Tropftrichter, Thermometer, aufgesetzter Kolonne mit Kondensator und Wasserabscheider versehen war, ließ man bei ca. 120 °C zu 296 g n-Butanol und 0,33 g konzentrierter Schwefelsäure unter Rühren innerhalb von 30 Minuten 84 g Diketen eintropfen. Die Dampfphase des siedenden Gemisches wurde im Kondensator niedergeschlagen, das Reaktionswasser abgetrennt und das Butanol in den Kolben zurückgeführt. Nach 2 Stunden ersetzte man die im Wasserabscheider gesammelte Menge von 46 g Butanol durch eine ebenso große Menge Frischbutanol. Während der Reaktion wurde ein leichter Stickstoffstrom durch die Apparatur geleitet. Nach 4 Stunden Reaktionszeit enthielt das Gemisch folgende Komponenten: 205 g n-Butanol, 93 g Acetessigsäurebutylester, 69 g Acetylketendibutylacetal, 4 g Unbekannte. Ließ man das Gemisch weiterreagieren, so stieg die Reaktionstemperatur allmählich an und das Verhältnis Acetylketendibutylacetal : Acetessigsäurebutylester verschob sich immer mehr zugunsten der Ketenkomponente. Nach 14 Stunden wurde eine Reaktionstemperatur von 130 °C erreicht, und es befanden sich in dem Gemisch 183 g n-Butanol, 33 g Acetessigsäurebutylester, 141 g Acetylketendibutylacetal, 11 g Unbekannte.

Aus dem Reaktionsgemisch ließ sich durch fraktionierte Destillation Acetylketendibutylacetal mit einer Reinheit von 99 % gewinnen.


**Beispiel 2**

In derselben Apparatur wie in Beispiel 1 ließ man bei ca. 140 °C zu 352 g n-Pentanol und 0,33 g konzentrierter Schwefelsäure unter Rühren innerhalb von 10 Minuten 84 g Diketen eintropfen. Der Wasseraustrag erfolgte in derselben Weise wie in Beispiel 1. Nach 2 Stunden bei einer Reaktionstemperatur von 148 °C enthielt das Gemisch folgende Komponenten: 233 g n-Pentanol, 81 g Acetessigsäurepentylester, 103 g Acetylketendipentylacetal, 9 g Unbekannte. Analog zu Beispiel 1 nahm mit fortschreitender Zeit und ansteigender Reaktionstemperatur der Acetylketendipentylacetal-Gehalt auf Kosten des Acetessigsäurepentylester-Anteils zu. Nach 13,5 Stunden enthielt das Gemisch bei 155 °C 197 g n-Pentanol, 23 g Acetessigsäurepentylester, 188 g Acetylketendipentylacetal, 16 g Unbekannte.

Aus dem Reaktionsgemisch ließ sich durch fraktionierte Destillation Acetylketendipentylacetal mit einer Reinheit von 99 % gewinnen.


**Beispiel 3**

In einem mit Rührer, Tropftrichter, Thermometer und aufgesetzter Kolonne mit Kondensator ausgestatteten 2 l-Vierhalskolben ließ man zu 1216 g Methylglykol und 1,6g konzentrierter Schwefelsäure unter Rühren im Verlauf von 20 Minuten 336 g Diketen eintropfen. Die Temperatur stieg dabei auf ca. 125 °C an. Um das entstehende Reaktionswasser in einfacher Weise zu entfernen, trug man in den Kolben Methylglykol mit einer Rate von 40 g/h ein, das verdampfte und in gleicher Menge am Kondensator der Apparatur abgezogen wurde. Die Reaktionstemperatur betrug konstant 125 °C. Nach 15 Stunden wurde die Reaktion beendet. Das Gemisch enthielt 960 g Methylglykol, 179 g Acetessigsäure-2-methoxyethylester und 134 g Acetylketendi-2-methoxyethylacetal.

Aus dem Gemisch ließ sich durch fraktionierte Destillation Acetylketendi-2-methoxyethylacetal mit einer Reinheit von 98 %gewinnen. Bestimmungswerte: Fp 10-15 °C; $d_4^{20}$ 1,0786; $n_D^{20}$ 1,4667

| Elementaranalyse | | | |
|---|---|---|---|
| berechnet: | 55,0 % C; | 8,3 % H; | 36,7 % O; |
| gefunden: | 55,1 % C; | 8,2 % H; | 36,0 % O. |


**Beispiel 4**

In derselben Apparatur wie in Beispiel 3 ließ man zu 1440 g Ethylglykol und 1,8 g konzentrierter Schwefelsäure unter Rühren im Verlauf von 1,5 Stunden 336 g Diketen zutropfen. Bei einer Temperatur von 140 bis 145 °C trug man in den Kolben zur Entfernung des entstehenden Reaktionswassers Ethylglykol mit einer Rate von 60 g/h ein, das verdampfte und in gleicher Menge am Kondensator der Apparatur flüssig abgezogen wurde. Nach 18 Stunden wurde die Reaktion beendet. Das Gemisch enthielt 1110 g Ethylglykol, 170 g Acetessigsäure-2-ethoxyethylester, 184 g Acetylketendi-2-ethoxyethylacetal.

Aus dem Gemisch ließ sich durch fraktionierte Destillation sowie fraktionierte Kristallisation Acetylketendi-2-ethoxyethylacetal mit einer Reinheit von 99 % gewinnen.

Bestimmungswerte: Fp 28,7 °C; $d_4^{30}$ 1,0250; $n_D^{32}$ 1,4564

| Elementaranalyse | | | | |
|---|---|---|---|---|
| berechnet: | 58,5 % C; | 8,9 % H; | 32,5 % O; |
| gefunden: | 58,2 % C; | 8,9 % H; | 32,4 % O. |

## Ansprüche

1. Acetale der Formel (I)

$CH_3\text{-}CO\text{-}CH = C(OR)_2$    (I)

wobei $R = -(CH_2)_n\text{-}OR'$ mit n = 2 oder 3 und $R' = C_1\text{-}C_5$-Alkyl ist.

2. Verfahren zur Herstellung von Acetalen der Formel (I)

$CH_3\text{-}CO\text{-}CH = C(OR)_2$    (I)

wobei $R = C_1\text{-}C_{12}$-Alkyl oder $R = -(CH_2)_n\text{-}OR'$ mit n = 2 oder 3 und $R' = C_1\text{-}C_5$-Alkyl ist, dadurch gekennzeichnet, daß man Diketen in Gegenwart eines sauren Katalysators mit einem Alkohol der Formel ROH umsetzt, wobei R die angegebene Bedeutung hat.

3. Verfahren nach Anspruch 2, dadurch gekennzeichnet, daß man pro Mol Diketen 1,5 bis 6 Mol Alkohol ROH einsetzt.

4. Verfahren nach Anspruch 2 oder 3, dadurch gekennzeichnet, daß man einen primären gesättigten Alkohol der Formel ROH mit $R = C_3\text{-}C_6$-Alkyl einsetzt.

5. Verfahren nach Anspruch 2 oder 3, dadurch gekennzeichnet, daß man einen Alkohol der Formel $R'O\text{-}(CH_2)_n\text{-}OH$ mit n = 2 und $R' = C_1\text{-}C_3$-Alkyl einsetzt.

6. Verfahren nach mindestens einem der Ansprüche 2 bis 5, dadurch gekennzeichnet, daß man bei einer Temperatur von 110 bis 170 °C arbeitet.

7. Verfahren nach mindestens einem der Ansprüche 2 bis 5, dadurch gekennzeichnet, daß man bei einer Temperatur von 130 bis 160 °C arbeitet.

8. Verfahren nach mindestens einem der Ansprüche 2 bis 7, dadurch gekennzeichnet, daß man als Katalysatoren Protonensäuren in einer Konzentration von 0,005 bis 5 Gew.-% verwendet.

9. Verfahren nach mindestens einem der Ansprüche 2 bis 7, dadurch gekennzeichnet, daß man als Katalysator Schwefelsäure in einer Konzentration von 0,05 bis 0,2 Gew.-% verwendet.

| | EINSCHLÄGIGE DOKUMENTE | | EP 89109311.4 | |
|---|---|---|---|---|
| **Kategorie** | **Kennzeichnung des Dokuments mit Angabe, soweit erforderlich, der maßgeblichen Teile** | | **Betrifft Anspruch** | **KLASSIFIKATION DER ANMELDUNG (Int. Cl.4)** |
| P,A<br><br>P,D,<br>A | EP - A1 - 0 273 378<br>(HOECHST)<br>  * Zusammenfassung *<br>  & DE-A1-3 644 661<br><br>---- | | 1,2 | C 07 C 49/255<br>C 07 C 45/57<br>C 07 C 45/87 |
| | | | | **RECHERCHIERTE SACHGEBIETE (Int. Cl.4)**<br><br>C 07 C 49/00<br>C 07 C 45/00 |

Der vorliegende Recherchenbericht wurde für alle Patentansprüche erstellt.

| Recherchenort | Abschlußdatum der Recherche | Prüfer |
|---|---|---|
| WIEN | 01-09-1989 | REIF |